# EUROPÄISCHE PATENTSCHRIFT

(11) **EP 3 594 330 B1**
(45) Veröffentlichungstag und Bekanntmachung des Hinweises auf die Patenterteilung: **21.04.2021**
(21) Anmeldenummer: 18182888.0
(22) Anmeldetag: 11.07.2018
(51) Int. Cl.: C12N 7/02

(54) **VERFAHREN ZUM ABTRENNEN VIRUSARTIGER PARTIKEL AUS EINER ZELLSUSPENSION**
METHOD FOR SEPARATING OF VIRUS-LIKE PARTICLES FROM A CELL SUSPENSION
PROCÉDÉ DE SÉPARATION DES PARTICULES VIROÏDES D'UNE SUSPENSION CELLULAIRE

(43) Veröffentlichungstag der Anmeldung: 15.01.2020
(73) Patentinhaber: Artes Biotechnology GmbH, 40764 Langenfeld (DE)
(72) Erfinder: Wetzel, David, 40723 Hilden (DE); Jenzelewski, Volker, 41464 Neuss (DE); Piontek, Michael, 42549 Velbert (DE)
(74) Vertreter: Zenz Patentanwälte Partnerschaft mbB

(56) Entgegenhaltungen:
- DAVID WETZEL ET AL: "Establishment of a yeast-based VLP platform for antigen presentation", MICROBIAL CELL FACTORIES, Bd. 17, Nr. 1, 5. Februar 2018 (2018-02-05), XP055511568, DOI: 10.1186/s12934-018-0868-0
- Pascale Brocke ET AL: "Recombinant Hepatitis B Vaccines: Disease Characterization and Vaccine Production : Novel Microbial and Eukaryotic Expression Systems" In: "Production of Recombinant Proteins : Novel Microbial and Eukaryotic Expression Systems", 4. November 2004 (2004-11-04), Wiley-VCH Verlag GmbH & Co. KGaA, Weinheim, FRG, XP055511571, ISBN: 978-3-527-31036-4 Seiten 319-359, DOI: 10.1002/3527603670.ch15, * Abbildung 15.12 * * Abschnitt 15.3.3.2 *
- MARIA ZAHID ET AL: "Assessing stability and assembly of the hepatitis B surface antigen into virus-like particles during down-stream processing", VACCINE, Bd. 33, Nr. 31, 1. Juli 2015 (2015-07-01), Seiten 3739-3745, XP055511574, AMSTERDAM, NL ISSN: 0264-410X, DOI: 10.1016/j.vaccine.2015.05.066
- GURRAMKONDA CHANDRASEKHAR ET AL: "Purification of hepatitis B surface antigen virus-like particles from recombinantPichia pastorisandin vivoanalysis of their immunogenic properties", JOURNAL OF CHROMATOGRAPHY B: BIOMEDICAL SCIENCES & APPLICATIONS, Bd. 940, 27. September 2013 (2013-09-27), Seiten 104-111, XP028758636, ISSN: 1570-0232, DOI: 10.1016/J.JCHROMB.2013.09.030
- NADIA HADIJI-ABBES ET AL: "Extraction and purification of hepatitis B virus-like M particles from a recombinant Saccharomyces cerevisiae strain using alumina powder", JOURNAL OF VIROLOGICAL METHODS, Bd. 187, Nr. 1, 1. Januar 2013 (2013-01-01), Seiten 132-137, XP055511575, NL ISSN: 0166-0934, DOI: 10.1016/j.jviromet.2012.09.023

## Beschreibung

Die Erfindung betrifft ein Verfahren zum Abtrennen virusartiger Partikel aus einer Zellsuspension von die virusartigen Partikel enthaltenden Wirtszellen, wobei die virusartigen Partikel wenigstens ein in eine Lipid-Doppelmembran eingebettetes Hüllprotein aufweisen, das zumindest einen Abschnitt umfasst, der einem kleinen Hüllprotein eines Virus der Familie Hepadnaviridae entspricht, wobei
a) die Wirtszellen aufgeschlossen werden, um eine die virusartigen Partikel enthaltende erste Suspension zu gewinnen,
b) ein die virusartigen Partikel enthaltender Überstand von der ersten Suspension abgetrennt wird,
c) dem von der ersten Suspension abgetrennten Überstand ein Adsorptionsmittel derart zugegeben wird, dass die virusartigen Partikel von dem Adsorptionsmittel adsorbiert werden,
d) das Adsorptionsmittel mit den adsorbierten virusartigen Partikeln abgetrennt wird,
e) die virusartigen Partikel von dem abgetrennten Adsorptionsmittel desorbiert werden, so dass sie in einen Überstand einer dabei gebildeten zweiten Suspension gelangen und
f) der die virusartigen Partikel enthaltende Überstand von der zweiten Suspension abgetrennt wird,
wobei anschließend die virusartigen Partikel aus dem abgetrennten Überstand abgetrennt und gereinigt werden.

Ein solches Verfahren ist beispielsweise bekannt aus S. Schaefer, M. Piontek, S.-J. Ahn, A. Papendieck, Z. A. Janowicz, I. Timmermans und G. Gelissen: "Recombinant hepatitis B vaccines - disease characterization and vaccine production", chapter 12.3.3, in G. Gellissen (ed): "Hansenula polymorpha - Biology and Applications", 2002, Wiley-VCH Verlag GmbH, Weinheim. Bei dem dort beschriebenen, der Fermentation nachgeschalteten Prozess (Downstream-Prozess) werden zunächst die Wirtszellen der Hefeart *Ogataea angusta* (*Hansenula polymorpha*) geerntet, die virusartige Partikel enthalten, wobei die virusartigen Partikel in eine Lipid-Doppelmembran eingebettete Hüllproteine HBsAg (Hepatitis-B-Oberflächen-Antigen) aufweisen. Es folgt ein Zellaufschlussverfahren mittels Mahlen in einer Glaskugelmühle oder mittels Hochdruckhomogenisierung. Im Ergebnis liegt eine erste Suspension vor, die nur noch wenige intakte Wirtszellen und die Bestandteile der zerstörten Wirtszellen, einschließlich der dabei freigesetzten virusartigen Partikel, enthält. Es folgt eine Zentrifugation, bei der ein Überstand abgetrennt und der weiteren Verarbeitung zugeführt wird. Der Überstand enthält die virusartigen Partikel, die in einem nachfolgenden Adsorptionsschritt an ein geeignetes Adsorptionsmittel spezifisch gebunden werden. Das Adsorptionsmittel ist so gewählt, dass der überwiegende Teil der virusartigen Partikel daran gebunden wird, während der größte Teil der übrigen Proteine der Wirtszellen im Überstand verbleibt. Die ungebundenen Proteine im Überstand werden entfernt, indem das Adsorptionsmittel mit den daran gebundenen virusartigen Partikeln gewaschen wird. Das gewaschene Adsorptionsmittel wird anschließend mit einem Desorptionsmittel resuspendiert, wobei sich der überwiegende Teil der virusartigen Partikel von dem Adsorptionsmittel löst. Der die virusartigen Partikel enthaltende Überstand wird abgetrennt und weiteren Reinigungsschritten zugeführt. Die weiteren Reinigungsschritte umfassen eine Ionenaustauschchromatographie und eine Ultrafiltration, der eine Cäsiumchlorid-Dichtegradienten-Zentrifugation folgt. An diese Ultrazentrifugation schließt sich eine Gelfiltrationschromatographie an, um das Cäsiumsalz aus dem Produkt zu entfernen.

Nachteilig bei dem bekannten Verfahren sind die relativ hohen Kosten, die insbesondere durch den Schritt der Cäsiumchlorid-Dichtegradienten-Zentrifugation mit nachfolgender Gelfiltrationschromatographie entstehen.

Es ist daher eine der Erfindung zugrunde liegende Aufgabe, ein Verfahren bereitzustellen, bei dem bei vergleichbarer Reinheit der hergestellten virusartigen Partikel auf eine nachfolgende Cäsiumchlorid-Dichtegradienten-Zentrifugation verzichtet werden kann.

Diese Aufgabe wird durch ein Verfahren mit den Merkmalen des Anspruchs 1 gelöst. Das heißt, bei einem Verfahren der eingangs genannten Art wird diese Aufgabe dadurch gelöst, dass im Schritt e) zum Desorbieren der virusartigen Partikel ein Desorptionspuffer zugegeben wird, der Desoxycholsäure und/oder wenigstens ein lösliches Salz der Desoxycholsäure enthält, und dass nach dem Schritt f):
g) dem abgetrennten Überstand ein lösliches Calciumsalz zugegeben wird, wobei ein ein Calciumsalz der Desoxycholsäure und die virusartigen Partikel enthaltender Niederschlag gebildet wird,
h) der gebildete Niederschlag abgetrennt und in eine dritte Suspension überführt wird und
i) die virusartigen Partikel aus der dritten Suspension abgetrennt und gereinigt werden.

Bei dem erfindungsgemäßen Verfahren zum Abtrennen virusartiger Partikel aus einer Zellsuspension von die virusartigen Partikel enthaltenden Wirtszellen werden zunächst in einem Schritt a) die Wirtszellen aufgeschlossen, um eine die virusartigen Partikel enthaltende erste Suspension zu gewinnen. Das Aufschließen der Wirtszellen kann mit irgendeinem hierzu geeigneten Verfahren erfolgen, wie sie auch im oben genannten Stand der Technik erwähnt sind. Die erste Suspension enthält somit sämtliche Bestandteile der Wirtszellen einschließlich der freigesetzten virusartigen Partikel, welche wenigstens ein in eine Lipid-Doppelmembran eingebettetes Hüllprotein aufweisen, das zumindest einen Abschnitt umfasst, der einem kleinen Hüllprotein eines Virus der Familie Hepadnaviridae entspricht. Das kleine Hüllprotein ist das kleinste von mehreren von diesen Viren gebildeten Hüllproteinen. Wenn hier davon die Rede ist, dass die virusartigen Partikel "wenigstens ein" Hüllprotein aufweisen, so bezieht sich dies auf die Art der Hüllproteine und bedeutet regelmäßig, dass eine Vielzahl von Molekülen dieses Hüllproteins in die Lipid-Doppelmembran der Partikel eingebettet sind. Üblicherweise sind eine Vielzahl von Hüllprotein-Molekülen, beispielsweise etwa 100 Hüllprotein-Moleküle, in die Lipid-Doppelmembran der virusartigen Partikel eingebettet. Außerdem schließt dies nicht aus, dass mehrere verschiedene Hüllproteine in die Lipid-Doppelmembran eingebettet sein können. Wenn außerdem davon gesprochen wird, dass das Hüllprotein "zumindest einen Abschnitt" umfasst, der einem kleinen Hüllprotein eines Virus der Familie Hepadnaviridae entspricht, so bedeutet dies im einfachsten Fall, dass das Hüllprotein aus dem kleinen Hüllprotein eines Virus der Familie Hepadnaviridae besteht. Es bedeutet aber im Allgemeinen, dass das Hüllprotein neben dem dem kleinen Hüllprotein entsprechenden Abschnitt weitere Abschnitte umfasst, die entweder den weiteren Abschnitten anderer Hüllproteine desselben Virus der Familie Hepadnaviridae entsprechen oder die Protein-Abschnitten anderer Organismen entsprechen. Darüber hinaus können auch mehrere verschiedene Abschnitte an den zumindest einen dem kleinen Hüllprotein entsprechenden Abschnitt angekoppelt sein. Die Zusammensetzung der Lipid-Doppelmembran entspricht der der Lipid-Doppelmembranen der Wirtszelle.

In einem zweiten Schritt b) wird ein die virusartigen Partikel enthaltender Überstand von der ersten Suspension abgetrennt. Beispielsweise erfolgt dieses Abtrennen durch die hier übliche Zentrifugation, wobei der gebildete Überstand, das Lysat, der weiteren Verarbeitung zugeführt wird.

Anschließend wird in einem dritten Schritt c) dem abgetrennten Überstand ein Adsorptionsmittel derart zugegeben, dass die virusartigen Partikel von dem Adsorptionsmittel adsorbiert werden. Beispielsweise wird ein Lysat in einer Pufferlösung mit einem geeigneten Adsorptionsmittel gemischt, wobei die Pufferlösung einen für eine Adsorption der virusartigen Partikel an dem Adsorptionsmittel geeigneten pH-Wert aufweist. Als Adsorptionsmittel kann beispielsweise pyrogenes oder gefälltes Siliciumdioxid oder Glasmilch oder auch eine anderer Stoff mit einer hydrophilen Oberfläche, die Silanolgruppen (-Si-OH) aufweist, verwendet werden. Bei diesem Schritt findet eine Adsorption der virusartigen Partikel an das Adsorptionsmittel statt. Diese Adsorption erfordert üblicherweise eine gewisse Zeit, so dass beispielsweise die Mischung aus dem Lysat und dem Adsorptionsmittel für eine gewisse Zeitdauer bei vorgegebenen Reaktionsbedingungen gerührt wird.

Anschließend wird im Schritt d) das Adsorptionsmittel mit den adsorbierten virusartigen Partikeln abgetrennt. Beispielsweise wird die das Adsorptionsmittel enthaltende Suspension zentrifugiert, der dabei gebildete Niederschlag, das so genannte "Pellet", in einer Waschlösung resuspendiert, die dabei gebildete Waschlösungssuspension anschließend zentrifugiert und der dabei gebildete Niederschlag ("Pellet"), der die gewaschenen Adsorptionsmittel-Partikel mit adsorbierten virusartigen Partikeln enthält, abgetrennt.

In einem nachfolgenden Schritt e) werden die virusartigen Partikel von dem abgetrennten Adsorptionsmittel desorbiert, so dass sie in einen Überstand einer dabei gebildeten zweiten Suspension gelangen. Dies erfolgt beispielsweise dadurch, dass ein Niederschlag oder Pellet des abgetrennten gewaschenen Adsorptionsmittels (mit adsorbierten virusartigen Partikeln) unter Zugabe eines Desorptionsmittels in einer Pufferlösung resuspendiert ("aufgelöst") wird, wobei die Pufferlösung einen für eine Desorption der virusartigen Partikel von dem Adsorptionsmittel geeigneten pH-Wert aufweist. Zum Desorbieren der virusartigen Partikel wird - in an sich bekannter Weise - ein Desorptionspuffer zugegeben, der als Detergens Desoxycholsäure und/oder wenigstens ein lösliches Salz der Desoxycholsäure enthält. Daneben kann der Desorptionspuffer übliche Puffer-Bestandteile, wie beispielsweise Borate und EDTA (Ethylendiamintetraessigsäure) enthalten.

Anschließend wird in einem Schritt f) der Überstand der zweiten Suspension, der nun die virusartigen Partikel enthält, von der zweiten Suspension abgetrennt, beispielsweise durch Zentrifugieren. In den nachfolgenden Schritten werden die virusartigen Partikel aus dem abgetrennten Überstand abgetrennt und gereinigt.

Insbesondere wird dazu in einem Schritt g) dem abgetrennten Überstand ein lösliches Calciumsalz zugegeben, wobei ein Niederschlag gebildet wird, der ein Calciumsalz der Desoxycholsäure und die virusartigen Partikel enthält. Bei diesem Schritt findet ein Ausfällen der Desoxycholsäure durch Zugabe von zweiwertigen Kationen, nämlich Calciumionen statt. Überraschenderweise zeigte sich hierbei, dass durch die Zugabe des löslichen Calciumsalzes, beispielsweise von Calciumchlorid, zusammen mit der Desoxycholsäure auch der überwiegende Teil der virusartigen Partikel in den Niederschlag gelangt, das heißt, gemeinsam ausgefällt wird.

Der dabei gebildete Niederschlag, der die virusartigen Partikel enthält, wird in einem Schritt h) abgetrennt und in eine dritte Suspension überführt. Beispielsweise wird der Niederschlag durch Zentrifugieren abgetrennt und das dabei gebildete "Pellet" in einer Pufferlösung resuspendiert. Der pH-Wert der Pufferlösung wird so eingestellt, dass sich die Desoxycholsäure löst, vorzugsweise auf einen leicht alkalischen Wert von z.B. 8,5. Die hier verwendete Pufferlösung enthält beispielsweise einen pH-Wert-Stabilisator, wie z.B. TRIS (Tris(hydroxymethyl)-aminomethan), einen Chelator, wie z.B. EDTA, und ein Salz, wie z.B. NaCl, zur Erhöhung der Ionenstärke.

Anschließend werden im Schritt i) die virusartigen Partikel aus der dritten Suspension abgetrennt und gereinigt. Dieses abschließende Abtrennen und Reinigen der virusartigen Partikel kann beispielsweise - wie auch bei dem oben genannten bekannten Verfahren - durch ein chromatographisches Verfahren und eine Filtration erfolgen.

Der entscheidende Vorteil des erfindungsgemäßen Verfahrens besteht darin, dass aufgrund der Reinheit der dritten Suspension, die wesentlich auf dem zwischengeschalteten Ausfäll-Schritt g) beruht, auf die nachfolgenden teuren Schritte der Cäsiumchlorid-Dichtegradienten-Zentrifugation und Größenausschluss-Chromatographie verzichtet werden kann.

Eine bevorzugte Weiterbildung des Verfahrens ist dadurch gekennzeichnet, dass im Schritt e) ein Desorptionspuffer zugegeben wird, der die Desoxycholsäure bzw. das lösliche Salz der Desoxycholsäure in einer Stoffmengenkonzentration zwischen 1 mmol/l und 10 mmol/l, vorzugsweise in einer Stoffmengenkonzentration von etwa 6 mmol/l, enthält. Diese im Stand der Technik für diesen Desorptionsschritt als solche bekannte Konzentration des Detergens oder Tensids Desoxycholsäure sichert eine ausreichende Desorption der virusartigen Partikel.

Dabei wird vorzugsweise im Schritt g) dem abgetrennten Überstand das lösliche Calciumsalz in einer Stoffmengenkonzentration zwischen 30 mmol/l und 100 mmol/l, vorzugsweise zwischen 40 mmol/l und 60 mmol/l, zugegeben. Es zeigte sich eine optimale Konzentration bei etwa 50 mmol/l; bei geringerer Konzentration sank der Anteil der ausgefällten virusartigen Partikel und höhere Konzentrationen hatten keine wesentliche Steigerung des ausgefällten Anteils zur Folge, sind aber weniger wirtschaftlich. Um ein Ausfällen der Desoxycholsäure bei Zugabe der Calciumsalzes zu erreichen, ist ein geeigneter pH-Wert im sauren Bereich einzustellen. Vorzugsweise wird hier ein pH-Wert zwischen 4 und 5,5 eingestellt. Dabei wird vorzugsweise eine Temperatur von 4 - 8°C eingestellt, wobei die Dauer des Ausfäll-Schritts beispielsweise bei etwa 6 Stunden liegt.

Vorzugsweise wird im Schritt e) zum Desorbieren der virusartigen Partikel von dem Adsorptionsmittel der die Desoxycholsäure und/oder das lösliche Salz der Desoxycholsäure enthaltende Desorptionspuffer in einer Menge zugegeben, die dem 0,1-fachen bis 4-fachen des Volumens des im Schritt b) von der die aufgeschlossenen Wirtszellen enthaltenden ersten Suspension abgetrennten Überstands entspricht. Beispielsweise wird der Desorptionspuffer in einer Menge zugegeben, die dem 0,25-fachen, d.h. einem Viertel, des Volumens des Überstands entspricht, der im Schritt b) von der ersten Suspension abgetrennt wurde. Vorzugsweise werden zwei Desorptionsschritte hintereinandergeschaltet, wobei jeweils der Desorptionspuffer in einer Menge zugegeben, die beispielsweise dem 0,125-fachen (einem Achtel) des Volumens des im Schritt b) von der ersten Suspension abgetrennten Überstands entspricht. Hierbei wird zuerst dem im Schritt d) abgetrennten Adsorptionsmittel ein erster Anteil des Desorptionspuffers zugegeben, dann der Überstand abgetrennt und aufbewahrt, dann dem abgetrennten Adsorptionsmittel ein zweiter Anteil des Desorptionspuffers zugegeben und wiederum der Überstand abgetrennt. Schließlich werden die beiden Mengen des jeweils abgetrennten Überstands zusammengeführt. Die eingesetzte Menge des Desorptionspuffers und der zweite Waschschritt steigern die Ausbeute.

Im Schritt g) können lösliche Calciumsalze verwendet werden, wie beispielsweise Calciumchlorid, Calciumascorbat, Calciumlactat, Calciumgluconat, Calciumlactatgluconat oder Calciumnitrat. Vorzugsweise wird im Schritt g) dem abgetrennten Überstand Calciumchlorid zugegeben.

Bei einer bevorzugten Ausführungsform wird im Schritt c) dem abgetrennten Überstand ein nichtionisches Tensid zugemischt, so dass (während der Adsorption) eine Konzentration des nichtionischen Tensids zwischen 0,006 g/ml und 0,01 g/ml, vorzugsweise eine Konzentration von etwa 0,008 g/ml, eingestellt wird. Vorzugsweise wird als nichtionisches Tensid Polysorbat 20 (Tween® 20; "Tween" ist eine eingetragene Marke der Croda Americas LLC) dem abgetrennten Überstand zugemischt. Dieses Detergens Polysorbat 20 befindet sich vorzugsweise bereits beim Zellaufschluss im Schritt a) in ersten Suspension. Die Zugabe im Schritt c) dient in diesem Fall der Erhöhung der Konzentration des Polysorbat 20. Überraschenderweise hatte die Zugabe des nicht-ionischen Tensids Polysorbat 20 einen Effekt hinsichtlich der Bindung von Wirtsproteinen und virusartigen Partikeln an die Oberfläche der als Adsorptionsmittel eingesetzten pyrogenen Kieselsäure; der Anteil der adsorbierten Wirtsproteine konnte gesenkt werden. Auf diese Weise wurde eine Steigerung der Reinheit erreicht. Das Einstellen einer Konzentration von etwa 0,008 g/ml ist bevorzugt; eine weitere Steigerung der Konzentration, insbesondere über 0,01 g/ml hinaus, führte zu keiner wesentlichen Verbesserung.

Bei einer vorteilhaften Weiterbildung des Verfahrens zum Abtrennen virusartiger Partikel aus einer Zellsuspension wird im Schritt d) das Adsorptionsmittel mit den adsorbierten virusartigen Partikeln abgetrennt, indem das Adsorptionsmittel mit den adsorbierten virusartigen Partikeln gewaschen wird, wobei der Waschlösung Harnstoff zugegeben wird. Überraschenderweise wirkte sich die Zugabe von Harnstoff zur Wasch-Lösung auf die Reinheit des Produkts aus, obgleich keine Auswirkungen einer Harnstoff-Zugabe auf die Adsorption festgestellt werden konnte. Grundsätzlich erhöht die Harnstoffzugabe zur Waschlösung den Anteil der abgewaschenen Wirtsproteine und den Anteil der abgewaschenen virusartigen Partikel, jedoch den Anteil der abgewaschenen Wirtsproteine überproportional.

Vorzugsweise wird hierbei das Adsorptionsmittel mit den adsorbierten virusartigen Partikeln gewaschen, indem in einem ersten Waschschritt der Waschlösung Harnstoff in einer Stoffmengenkonzentration zwischen 2 mol/l und 6 mol/l, vorzugsweise in einer Stoffmengenkonzentration von etwa 4 mol/l, zugegeben wird und in einem zweiten Waschschritt mit einer wässrigen Natriumchloridlösung gewaschen wird. Wie bereits erwähnt, erhöht die Harnstoffzugabe zur Waschlösung den Anteil der abgewaschenen Wirtsproteine und den Anteil der abgewaschenen virusartigen Partikel, jedoch den Anteil der abgewaschenen Wirtsproteine überproportional. Bei einer Harnstoff-Stoffmengenkonzentration zwischen 2 mol/l und 6 mol/l, vorzugsweise von etwa 4 mol/l, ist dieser überproportionale Anteil besonders hoch. Der zweite Waschschritt dient der Verringerung des Harnstoff-Übertrags in den Desorptionsansatz.

Bei einer Ausführungsform der Erfindung wird im Schritt h) der gebildete Niederschlag in eine dritte Suspension überführt, indem der Niederschlag in einer schwach basischen Pufferlösung aufgelöst wird. Der pH-Wert ist so gewählt, dass die Desoxycholsäure des Niederschlags in Lösung geht. Die im Niederschlag enthaltenen virusartigen Partikel treten ebenfalls in die hier wegen ihrer Klarheit auch als "Lösung" bezeichnete dritte Suspension über. Der pH-Wert wird vorzugsweise auf einen leicht alkalischen Wert von etwa 8,5 eingestellt. Die hier verwendete Pufferlösung enthält beispielsweise einen pH-Wert-Stabilisator, wie z.B. TRIS-Hydrochlorid (Tris(hydroxymethyl)-aminomethan-Hydrochlorid), einen Chelator, wie z.B. EDTA, und ein Salz, wie z.B. NaCl, zur Erhöhung der Ionenstärke.

Bei einer bevorzugten Weiterbildung des Verfahrens zum Abtrennen virusartiger Partikel aus einer Zellsuspension werden im Schritt i) die virusartigen Partikel mit Hilfe eines chromatographischen Verfahrens abgetrennt. Vorzugsweise wird dem chromatographischen Verfahren noch eine Filtration zur Beseitigung von Partikeln (beispielsweise 0,45µm-Filtration) vorgeschaltet, die die Chromatographie-Matrix verstopfen könnten. Ferner schließen sich bei einer bevorzugten Ausführungsform diesem Chromatographie-Schritt noch eine kombinierte Diafiltration zur Entsalzung und zum Pufferaustausch und eine Ultrafiltration zur Konzentrierung an.

Bei einer bevorzugten Ausführungsform des Verfahrens wird als chromatographisches Verfahren eine Anionen-Austausch-Chromatographie durchgeführt, wobei dem Eluenten ein Tensid zugemischt wird. Dies dient insbesondere dem Entfernen der zunächst im Schritt g) ausgefällten, dann aber durch Wiederauflösung im Schritt h) in der Suspension verbliebenen Desoxycholsäure. Hierbei wird im Schritt i) vorzugsweise dem Eluenten als Tensid Polysorbat 20 (Tween® 20) zugemischt. Dies erhöht die Ausbeute.

Bei einer alternativen Ausführungsform des Verfahrens wird als chromatographisches Verfahren anstelle der AnionenAustausch-Chromatographie eine multimodale Chromatographie durchgeführt. Im Unterschied zu der Anionen-Austausch-Chromatographie werden hier die virusartigen Partikel nicht gebunden, was das Verfahren beschleunigt. Da nicht eluiert werden muss, ergibt sich außerdem eine Materialeinsparung.

Bei einer Ausführungsform des Verfahrens zum Abtrennen virusartiger Partikel aus einer Zellsuspension von die virusartigen Partikel enthaltenden Wirtszellen weisen die virusartigen Partikel ein in die Lipid-Doppelmembran eingebettetes Hüllprotein auf, das zumindest einen Abschnitt umfasst, der dem kleinen Hüllprotein (s-HBsAg) eines humanen Hepatitis-B-Virus entspricht.

Bei einer bevorzugten Ausführungsform des Verfahrens zum Abtrennen virusartiger Partikel aus einer Zellsuspension von die virusartigen Partikel enthaltenden Wirtszellen weisen die virusartigen Partikel ein in die Lipid-Doppelmembran eingebettetes Hüllprotein auf, das zumindest einen Abschnitt umfasst, der dem kleinen Hüllprotein (s-dHBsAg) eines Enten-Hepatitis-B-Virus entspricht.

Vorzugsweise weisen die virusartigen Partikel ein Hüllprotein auf, das ein Hybridprotein mit einem dem kleinen Hüllprotein eines Virus der Familie Hepadnaviridae entsprechenden Abschnitt und wenigstens einem ein Zielepitop bildenden Abschnitt ist. Dies ermöglicht die Schaffung virusartiger Partikel mit wählbaren Epitopen, die Teil eines gewünschten Antigens sind.

Bei einer Weiterbildung diese Ausführungsform weist das Hybridprotein wenigstens zwei jeweils ein Zielepitop bildende Abschnitte auf. Dies vereinfacht die Schaffung virusartiger Partikel, die an ihrer Oberfläche verschiedene Epitope verschiedener Antigene präsentieren.

Bei den Ausführungsformen, bei denen das Hüllprotein ein Hybridprotein mit einem dem kleinen Hüllprotein eines Virus der Familie Hepadnaviridae entsprechenden Abschnitt und wenigstens einem ein Zielepitop bildenden Abschnitt ist, entspricht das wenigstens eine Zielepitop vorzugsweise einem Epitop eines Virus einer Gruppe, die Bovine Virusdiarrhoe-Viren, West-Nil-Viren und Schweinepest-Viren umfasst. Dies ermöglicht die kostengünstige Herstellung von Impfstoffen auf der Basis virusartiger Partikel.

Bei dem erfindungsgemäßen Verfahren zum Abtrennen virusartiger Partikel aus einer Zellsuspension von die virusartigen Partikel enthaltenden Wirtszellen ist es bevorzugt, dass die Wirtszellen Zellen eines rekombinanten Hefestamms der Ordnung Saccharomycetales (Echte Hefen) oder der Gattung Schizosaccharomyces (Spalthefen) sind. Vorzugsweise sind die Wirtszellen Zellen eines rekombinanten Stamms der *Ogataea angusta (Hansenula polymorpha).* Diese Hefe-Wirtsorganismen vereinigen die Vorteile einer einfachen genetischen Manipulation und einer eukaryotischen post-translationalen Modifikation mit einer hohen Produktivität und einem kostengünstigen Fermentationsverfahren.

Vorteilhafte und/oder gegenwärtig bevorzugte Ausführungsformen der Erfindung sind in den Unteransprüchen gekennzeichnet.

Nachfolgend wird die Erfindung anhand von in den Zeichnungen veranschaulichten bevorzugten Ausführungsformen des erfindungsgemäßen Verfahrens im Detail beschrieben. In den Zeichnungen zeigen:
Figur 1 eine schematische Darstellung eines bekannten Verfahrens zum Abtrennen virusartiger Partikel aus einer Zellsuspension von Wirtszellen und
Figur 2 eine schematische Darstellung einer ersten Ausführungsform des erfindungsgemäßen Verfahrens zum Abtrennen virusartiger Partikel aus einer Zellsuspension von Wirtszellen.

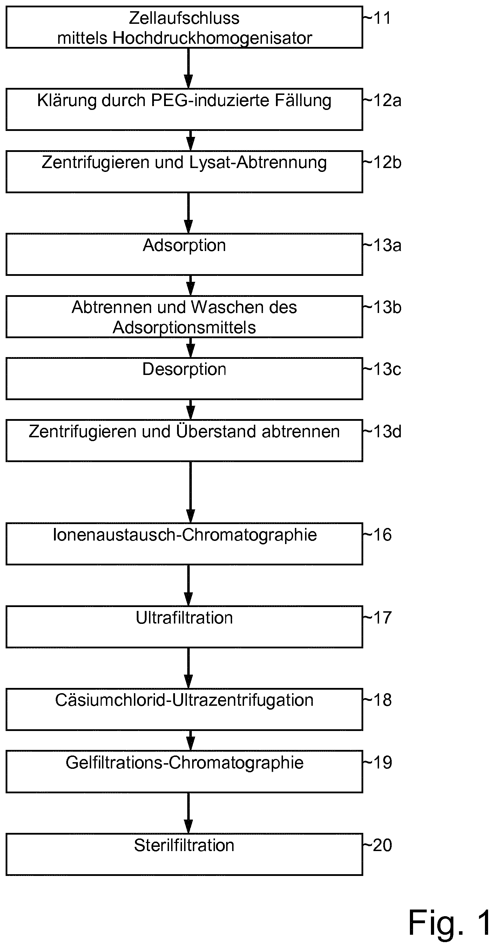

Das in Figur 1 dargestellte, bekannte Verfahren zum Abtrennen virusartiger Partikel aus einer Zellsuspension von Wirtszellen ist Teil eines aus dem eingangs genannten Artikel von Schaefer et al.: "Recombinant hepatitis B vaccines - disease characterization and vaccine production", chapter 12.3.3, in G. Gellissen (ed): "Hansenula polymorpha - Biology and Applications", 2002, Wiley-VCH Verlag GmbH, Weinheim, bekannten Downstream-Prozesses. Der Downstream-Prozess beginnt mit einer Ernte von Wirtszellen der der Hefeart *Ogataea angusta* (*Hansenula polymorpha*), die virusartige Partikel enthalten, wobei die virusartigen Partikel in eine Lipid-Doppelmembran eingebettete Hüllproteine HBsAg (Hepatitis-B-Oberflächen-Antigen) aufweisen. Das Kulturmedium wird dabei durch einen Puffer ersetzt, der für einen nachfolgenden Zellaufschluss geeignet ist. Daran schließen sich das Abtrennen der virusartigen Partikel aus einer Zellsuspension von geernteten Wirtszellen und die Reinigung der virusartigen Partikel an.

Bei dem bekannten Verfahren werden die Zellen zunächst mittels Hochdruckhomogenisierung aufgeschlossen (Schritt 11). Im Ergebnis liegt eine erste Suspension vor, die nur noch wenige intakte Wirtszellen und die Bestandteile der zerstörten Wirtszellen, einschließlich der dabei freigesetzten virusartigen Partikel, enthält. Die erste Suspension weist ein Puffersystem auf, das u. a. einen Inhibitor der intrazellularen Proteasen und Detergenzien enthält. Es folgen eine Klärung der Suspension durch Ausfällen mittels Zugabe von Polyethylenglykol (PEG) (Schritt 12a) und eine Hochgeschwindigkeitszentrifugation (Schritt 12b), wobei der Überstand abgetrennt und der weiteren Verarbeitung zugeführt wird. Der Überstand enthält die virusartigen Partikel, die in einem nachfolgenden Adsorptionsschritt an ein geeignetes Adsorptionsmittel (auch als Matrix bezeichnet) spezifisch gebunden werden (Schritt 13a). Das Adsorptionsmittel ist so gewählt, dass der überwiegende Teil der virusartigen Partikel daran gebunden wird, während der größte Teil der übrigen Proteine der Wirtszellen im Überstand verbleibt. Die ungebundenen Proteine im Überstand werden entfernt, indem das Adsorptionsmittel mit den daran gebundenen virusartigen Partikeln gewaschen wird (Schritt 13b). Das gewaschene Adsorptionsmittel wird anschließend mit einem Desorptionsmittel resuspendiert, wobei sich der überwiegende Teil der virusartigen Partikel von dem Adsorptionsmittel löst (Schritt 13c). Der die virusartigen Partikel enthaltende Überstand wird mittels Zentrifugation abgetrennt (Schritt 13d) und weiteren Reinigungsschritten zugeführt. Die weiteren Reinigungsschritte umfassen eine Ionenaustauschchromatographie (Schritt 16) und eine Ultrafiltration (Schritt 17), der eine Cäsiumchlorid-Dichtegradienten-Ultrazentrifugation (Schritt 18) zum Abtrennen restlicher Verunreinigungen durch Bestandteile der Wirtszellen folgt. An diese Ultrazentrifugation schließt sich eine Gelfiltrationschromatographie (Schritt 19) an, um das Cäsiumsalz aus dem Produkt zu entfernen. Gemäß der Veröffentlichung (Tabelle 12.5) wurde bei diesem Downstream-Prozess für die virusartigen Partikel HBsAg bei Wirtszellen der Hefeart *Ogataea angusta* (*Hansenula polymorpha*) eine Reinheit von mehr als 95 % (nach SDS/PAGE) erreicht, wobei im Produkt ein Rest-Cäsiumgehalt von weniger als 10 µg pro 20 µg Protein verbleibt.

Bei einer Abwandlung des aus dem Stand der Technik bekannten Verfahrens wurden mit Hilfe eines rekombinanten Hefestamms virusartige Partikel erzeugt, die in eine Lipid-Doppelmembran eingebettete Hüllprotein-Moleküle aufweisen, die einen ersten Abschnitt umfassen, der dem kleinen Hüllprotein des Enten-Hepatitis-B-Virus (dHBsAg) entsprechen, und die einen zweiten Abschnitt aufweisen, der ein aus dem Glykoprotein E2 des bovinen Virusdiarrhoe-Virus (BVDV - bovine viral diarrhea virus) abgeleitetes Antigen präsentiert. Der rekombinante Hefestamm exprimiert somit ein Fusionsprotein, das an seinem C-Ende das partikelbildende Hüllprotein dHBsAg aufweist, und das an seinem N-Ende das BVDV-E2-Protein (Genbank Zugriffsnummer: AEV54362.1) aufweist. Zunächst wurden die virusartigen Partikel nach einem Verfahren abgetrennt und gereinigt, das im Wesentlichen dem o.g. bekannten Verfahren entspricht. Dies dient einem Vergleich mit dem anschließend beschriebenen erfindungsgemäßen Verfahren.

Nach der Ernte der die virusartigen Partikel enthaltenden Wirtszellen wurden sechs Zyklen einer Hochdruckhomogenisierung bei 65±1g DCW/l in einem Zellaufschlusspuffer (25 mmol/l Natriumphosphat, 2 mmol/l EDTA, pH = 7,9) ausgeführt, der 0,07g Polysorbat 20 pro 1 g DCW (entspricht 0,46 %(w/v) Polysorbat 20) und 2 mmol/l des Protease-Inhibitors Phenylmethylsulfonylfluorid (PMSF) enthält. Das Lysat wurde direkt zentrifugiert (17.000 g, 4°C, 30 min) und der Überstand ("APV-SN") wurde weiterverarbeitet. Auf die aus dem o.g. Stand der Technik bekannte Klärung des Lysats durch Zugabe von PEG₆₀₀₀ und NaCl wurde verzichtet. Dem Überstand wurde als Adsorptionsmittel pyrogenes Siliciumdioxid (Aerosil 380 V, Evonik) zugegeben (15 g/l), und das Gemisch wurde über Nacht bei 4°C auf einen Magnetrührer der Adsorption überlassen. Das Adsorptionsmittel (mit den adsorbierten Partikeln) wurde dann abgetrennt und mit einer Kochsalzlösung (77 mmol/l) gewaschen, wobei das Volumen der Waschlösung auf das Volumen des Überstands APN-SN volumennormiert wurde. Anschließend wurde das Produkt durch Zugabe eines Desorptionspuffers (10 mmol/l Dinatriumtetraborat-Decahydrat, 2 mmol/l EDTA und 6 mmol/l Natriumdesoxycholat (DOC), pH = 9,1) unter Verwendung eines Viertels des Volumens des Überstands APV-SN desorbiert (eine Stunde bei 25°C gerührt). Die Suspension wurde anschließend zentrifugiert (17.000 g, 18°C, 30 min), wonach der Überstand (Desorbat) gefiltert wurde (0,45µm), um Siliciumdioxid-Teilchen zu entfernen. Dem gefilterten Desorbat wurde dann ein 50 mmol/l-TRIS-Hydrochlorid-Puffer (pH = 8,5, ∼8 mS/cm) hinzugefügt und eine Anionenaustausch-Chromatographie durchgeführt. Das Produkt wurde mit 0,5 mol/l NaCl in 50 mmol/l-TRIS-Hydrochlorid -Puffer (pH = 8,5) verdünnt und durch Diafiltration entsalzen sowie nachfolgend durch eine Ultrafiltration konzentriert. Das Retentat wurde einer Dichtegradienten-Ultrazentrifugation (1,5 mol/l Cäsiumchlorid, 65 Std. bei 48.400 rpm, 4°C) zugeführt. An diese Ultrazentrifugation schloss sich eine Größenausschluss-Chromatographie an, um das Cäsiumsalz aus dem Produkt zu entfernen. Abschließend folgte eine Sterilfiltration (0,45µm).

Bei diesem dem herkömmlichen Verfahren ähnlichen Abtrennungs- und Reinigungsverfahren wurden erwartungsgemäß Ergebnisse erzielt, die dem bekannten Verfahren entsprachen. Dies zeigt, dass die Reinigungsverfahren für die HBsAg-Partikel und die E2-BVDB-dHBsAg-Partikel vergleichbar sind. Erwartungsgemäß lassen sich die Ergebnisse des nachfolgend beschriebenen erfindungsgemäßen Reinigungsverfahrens auf eine Gruppe virusartiger Partikel übertragen, die wenigstens ein in eine Lipid-Doppelmembran eingebettetes Hüllprotein aufweisen, das zumindest einen Abschnitt umfasst, der einem kleinen Hüllprotein eines Virus der Familie Hepadnaviridae entspricht. Bei dem dem bekannten Verfahren entsprechenden Reinigungsverfahren ergab sich nach den Adsorptions-/Desorptions-Schritten (entsprechen den Schritten 13a - 13d gemäß Figur 1) eine Reinheit von 20 %, nach den Schritten der IonenaustauschChromatographie und Ultrafiltration (Schritte 16 und 17 des bekannten Verfahrens gemäß Figur 1) eine Reinheit von 29 %, nach den Schritten der Ultrazentrifugation mit anschließender Gelfiltrations-Chromatographie (Schritte 18 und 19 des bekannten Verfahrens) eine Reinheit von 90 % und nach der abschließenden 0,45µm-Sterilfiltration eine Reinheit von weiterhin 90 %. An diesen Ergebnissen erkennt man, dass zum Erzielen der gewünschten Reinheit die Cäsiumchlorid-Ultrazentrifugation einen wesentlichen Beitrag leistet, so dass ein Verzicht auf diesen Schritt zur Senkung der Kosten nicht oder zumindest nicht ohne erhebliche Modifikation der übrigen Reinigungsschritte ausführbar erscheint.

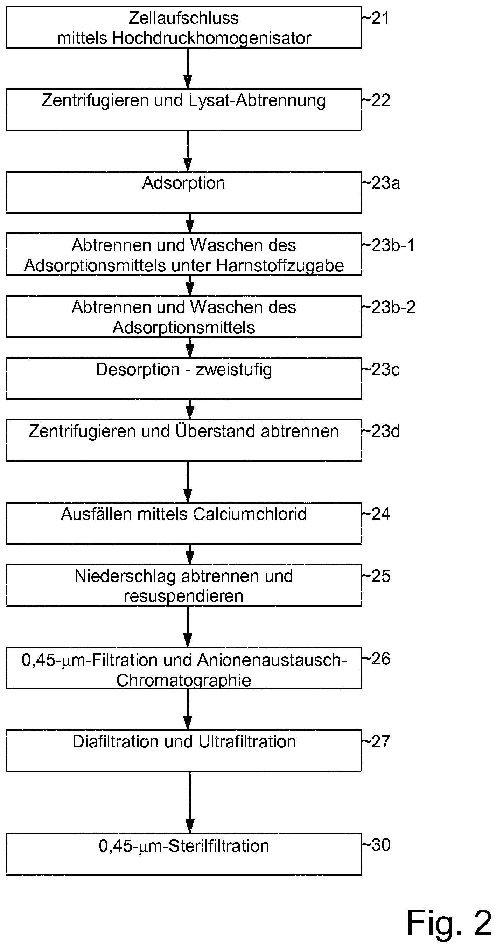

Figur 2 zeigt den schematischen Ablauf eines bevorzugten Ausführungsbeispiels des erfindungsgemäßen Verfahrens zum Abtrennen virusartiger Partikel aus einer Zellsuspension. Der Zellaufschluss mittels Hochdruckhomogenisator (Schritt 21) und der Schritt des Zentrifugierens und der Lysat-Abtrennung (Schritt 22) werden wie bei dem zuvor beschriebenen Verfahren durchgeführt.

Die Adsorptions-/Desorptions-Schritte (Schritte 23a - 23d) werden wie folgt modifiziert. Zunächst wurden verschiedene Bedingungen getestet, um während der Adsorption eine Steigerung der Reinheit zu erzielen. Überraschenderweise zeigte sich, dass die Zugabe des nicht-ionischen Detergens Polysorbat 20 (Tween® 20) Auswirkungen hinsichtlich der Bindung von Wirtsproteinen und der virusartigen Partikel an die ionische Oberfläche (SilanolGruppen) des pyrogenen Siliciumdioxids (Aerosil) hatte. Hierbei wurde berücksichtigt, dass sich dieses Detergens bereits beim Zellaufschluss in der Probe befindet (Zellaufschlusspuffer). In verschiedenen Test wurde die Konzentration stufenweise von 0,004 g/ml (kein zusätzliches Polysorbat 20) auf bis zu 0,014 g/ml gesteigert. Bei Konzentrationen bis zu etwa 0,008 g/ml zeigte sich eine Verringerung des Anteils der an das pyrogene Siliciumdioxid gebundenen Wirtsproteine im Vergleich zu den gebundenen virusartigen Partikeln. Bei weiterer Steigerung ab etwa 0,01 g/ml zeigten sich keine weiteren Verbesserungen. Deshalb wird bei der bevorzugten Ausführungsform des erfindungsgemäßen Verfahrens ein Anteil von 0,008 g/ml Polysorbat 20 im Adsorptionspuffer gewählt.

Dem bekannten Schritt des Waschens mit Kochsalzlösung (Schritt 23b-2) wird ein weiterer Waschschritt (Schritt 23b-1) vorgeschaltet. Zunächst wurden verschiedene Zusätze zu der Waschlösung hinsichtlich einer Steigerung der Reinheit des Produkts getestet. Überraschenderweise hatte die Zugabe von Harnstoff zur Waschlösung eine Auswirkung auf die Reinheit des Produkts, obgleich diese Substanz zuvor bei der Optimierung der Adsorption keinen Effekt zeigte. Getestet wurden Harnstoffkonzentrationen von bis zu 8 mol/l. Je höher die Harnstoffkonzentration gewählt wurde, desto mehr Wirtsproteine aber auch virusartige Partikel wurden vom pyrogenen Siliciumdioxid abgewaschen. Bei etwa 4 mol/l Harnstoff war der Anteil der abgewaschenen Wirtsproteine überproportional im Vergleich zum Anteil der abgewaschenen virusartigen Partikel. Deshalb wird bei der bevorzugten Ausführungsform des erfindungsgemäßen Verfahrens ein Anteil von 4 mol/l Harnstoff in einem 50 mmol/l-Natriumphosphat-Puffer (pH = 7,0) gewählt. In dem zweiten Waschschritt wird - wie bei dem bekannten Waschschritt - eine wässrige 77 mmol/l-NaCl-Lösung gewählt. Jedem Waschschritt geht ein Abtrennen des Adsorptionsmittels durch Zentrifugieren voraus, wonach das abgetrennte Adsorptionsmittel in der jeweiligen Waschlösung resuspendiert wird. Nach dem zweiten Waschschritt folgt wiederum ein Abtrennen des gewaschenen Adsorptionsmittels durch Zentrifugieren.

Auch der nachfolgende Schritt der Desorption (Schritt 23c) wird gegenüber dem bekannten Desorptionsschritt (Schritt 13c gemäß Figur 1) modifiziert. Dem durch Zentrifugieren abgetrennten gewaschenen Adsorptionsmittel wird ein Desorptionspuffer zugegeben. Dabei wurde festgestellt, dass bei höherem Volumen des Desorptionspuffers die Produktausbeute steigt. Deshalb wurde das Volumen des Desorptionspuffers gesteigert und ein zweiter Desorptionsschritt eingeführt. Während bei dem eingangs genannten Verfahren, das dem herkömmlichen Verfahren entspricht, ein Desorptionspuffer in einem Volumen zugegeben wurde, das einem Viertel des Volumens des Überstands APV-SN entspricht, wird bei dem modifizierten Desorptionsschritt dem abgetrennten Adsorptionsmittel ein erster Anteil des Desorptionspuffers in einem Volumen zugegeben, der einem Achtel des Volumens des Überstands APV-SN entspricht, dann der Überstand abgetrennt und aufbewahrt, dann dem abgetrennten Adsorptionsmittel ein zweiter Anteil des Desorptionspuffers zugegeben, der wiederum einem Achtel des Volumens des Überstands APV-SN entsprach, und wiederum der Überstand abgetrennt. Schließlich werden die beiden Mengen des jeweils abgetrennten Überstands zusammengeführt. Im Übrigen entspricht bei der bevorzugten Ausführungsform des erfindungsgemäßen Verfahrens der verwendete Desorptionspuffer dem oben beschriebenen Desorptionspuffer, der bei dem dem herkömmlichen Verfahren entsprechenden Verfahren verwendet wird.

Der zur Weiterverarbeitung nach dem Desorptionsschritt gewonnene Überstand enthält neben den abzutrennenden virusartigen Partikeln und unerwünschten Bestandteilen der Wirtszellen (insbesondere Wirtsproteinen) auch die Desoxycholsäure-Ionen des Desorptionspuffers. Erfindungsgemäße werden diese durch Zugabe zweiwertiger Kationen, beispielsweise von Calciumionen, ausgefällt. Dies erfolgt vorzugsweise im sauren Bereich bei einem pH-Wert zwischen 4 und 5,5. Da es ein Ziel der erfindungsgemäßen Verbesserung des Reinigungsverfahrens ist, die Kosten gering zu halten, werden als zweiwertige Kationen Calciumionen eingesetzt. Überraschenderweise zeigt sich, dass durch die Zugabe der Calciumionen nicht nur die Desoxycholsäure (DOC) ausgefällt wird, sondern zugleich auch die virusartigen Partikel ebenfalls mit ausgefällt werden. Sowohl die DOC als auch die virusartigen Partikel gelangen hierbei in einen Niederschlag (auch als Pellet bezeichnet), während ein großer Teil der im Produkt nicht erwünschten Bestandteile der Wirtszellen im Überstand verbleibt. Die Calciumionen werden vorzugsweise in Form von Calciumchlorid zugeführt, wobei beispielsweise unter Verwendung einer 1 mol/l-CaCl₂-Lösung eine Konzentration von 50 mmol/l im Desorptionspuffer eingestellt wird. Der Desorptionspuffer wirkt beispielsweise für eine Zeit von etwa sechs Stunden bei einer Temperatur zwischen 4 und 8°C ein.

Anschließend wird die Suspension zentrifugiert (17.000 g, 30 min, 18°C) und das so gewonnene Pellet wieder aufgelöst, wobei die darin enthaltenen virusartigen Partikel in eine Suspension überführt werden (Schritt 25 gemäß Figur 2). Das Auflösen der Pellets erfolgt in einem schwach alkalischen Puffer, wobei ein pH-Wert zwischen 7,5 und 8,5 die Löslichkeit der Desoxycholsäure verbessert. Der verwendete Puffer enthält beispielsweise 15 mmol/l TRIS-Hydrochlorid als pH-Wert-Stabilisator, 100 mmol/l Natriumchlorid zur Erhöhung der Ionenstärke und 20 mmol/l EDTA als Chelator. Vorzugsweise wird ein pH-Wert von 8,5 eingestellt. Anstelle des EDTA wurde EGTA getestet, wobei EDTA bessere Ergebnisse erbrachte als EGTA, obgleich EGTA in der Literatur als besserer Komplexbildner für Ca²⁺-Ionen beschrieben ist.

In einem nachfolgenden Schritt (Schritt 26) wird die so hergestellte Suspension bei 0,45pm gefiltert und dann einer Anionenaustausch-Chromatographie zugeführt. Die Filterung dient dem Entfernen von Partikeln, die die nachfolgende Chromatographie verstopfen können. Die Anionenaustausch-Chromatographie (AIEC) ist insbesondere zum Entfernen der noch in der Probe verbliebenen Desoxycholsäure erforderlich. Hierbei wird bei dem bevorzugten Verfahren beispielsweise ein Elutionspuffer verwendet, der 500 mmol/l NaCl und 0,1 Vol-% Polysorbat 20 in 50 mmol/l TRIS-Hydrochlorid-Puffer (pH = 8,5) enthält. Die Zugabe des nichtionischen Tensids Polysorbat 20 erhöht die Ausbeute.

Bei einem alternativen Ausführungsbeispiel des erfindungsgemäßen Verfahrens könnte die Anionenaustausch-Chromatographie im Schritt 26 ersetzt werden durch eine multimodale Chromatographie, die die Prinzipien verschiedener Chromatographiearten miteinander kombiniert, wie beispielsweise Gelfiltration mit Ionenaustauschchromatographie und hydrophober Interaktionschromatographie. Die multimodale Chromatographie nutzt im Vergleich zur Anionenaustuasch-Chromatographie ein anderes Prinzip: Die virusartigen Partikel werden nicht an die Chromatographie-Matrix gebunden. Nur die im Vergleich zu den virusartigen Partikeln verhältnismäßig kleinen Verunreinigungen (z.B. Wirtszellproteine) dringen in die Kügelchen der Matrix der multimodalen Chromatographie ein und binden entweder an die Liganden oder werden durch diffusionsbedingte Zeitverzögerung beim Durchfließen der Säule von den virusartigen Partikeln abgetrennt. Dadurch kann sich in Abhängigkeit von der Art der kontaminierenden Wirtszellproteine ein Vorteil gegenüber der Anionenaustausch-Chromatographie hinsichtlich der Reinheit der virusartigen Partikel ergeben. Außerdem ist diese Prozedur schneller, da die virusartigen Partikel nicht gebunden werden und dementsprechend nicht eluiert zu werden braucht, woraus sich außerdem ein Einsparungspotential bei den Materialien ergibt, weil beim Elutionsschritt der Anionenaustausch-Chromatographie eine nicht unerhebliche Menge NaCl eingesetzt werden muss (500 mmol/l bis 1 mol/l).

An die Chromatographie des Schritts 26 schließen sich eine Diafiltration und eine Ultrafiltration an, wie sie auch bereits im oben genannten bekannten Verfahren verwendet werden, das dem herkömmlichen Verfahren entspricht. Abschließend wird eine 0,45 µm-Sterilfiltration durchgeführt.

Bei dem erfindungsgemäßen Verfahren zeigte sich in den durchgeführten Versuchen bereits nach dem Schritt des Ausfällens mittels Calciumchlorid und dem Wieder-Auflösen des dabei gebildeten Pellets eine Reinheit der virusartigen Partikel von 72 %. Dies entspricht etwa der Reinheit, die bei dem herkömmlichen Verfahren nach der Ultrazentrifugation erreicht wird. Aufgrund dieses überraschenden Ergebnisses kann der teure Schritt der Cäsiumchlorid-Ultrazentrifugation mit nachfolgender Gelfiltrations-Chromatographie entfallen. In den durchgeführten Versuchen betrug die Reinheit nach der Ultrafiltration (Schritt 27) und nach der abschließenden Sterilfiltration 84 %. Bei dem alternativen Verfahren, bei dem anstelle der Anionenaustausch-Chromatographie eine multimodale Chromatographie eingesetzt wurde, betrug die Reinheit nach der Ultrafiltration (Schritt 27) 83 %. Nach der abschließenden 0,45µm-Filtration wurde eine Reinheit von 94 % festgestellt. Allerdings wurde ein erheblicher Verlust des Produkts bei der abschließenden Filtration festgestellt, was auf eine erhöhte Ausbildung von Clustern oder Aggregaten der virusartigen Partikel zurückzuführen sein könnte. Möglicherweise entfernen die verwendeten multimodalen Oktylamin-Liganden Bestandteile, wie beispielsweise Lipide, welche für die Ausbildung der virusartigen Partikel wesentlich sind.

## Patentansprüche

1. Verfahren zum Abtrennen virusartiger Partikel aus einer Zellsuspension von die virusartigen Partikel enthaltenden Wirtszellen, wobei die virusartigen Partikel wenigstens ein in eine Lipid-Doppelmembran eingebettetes Hüllprotein aufweisen, das zumindest einen Abschnitt umfasst, der einem kleinen Hüllprotein eines Virus der Familie Hepadnaviridae entspricht, wobei
a) die Wirtszellen aufgeschlossen werden, um eine die virusartigen Partikel enthaltende erste Suspension zu gewinnen,
b) ein die virusartigen Partikel enthaltender Überstand von der ersten Suspension abgetrennt wird,
c) dem von der ersten Suspension abgetrennten Überstand ein Adsorptionsmittel derart zugegeben wird, dass die virusartigen Partikel von dem Adsorptionsmittel adsorbiert werden,
d) das Adsorptionsmittel mit den adsorbierten virusartigen Partikeln abgetrennt wird,
e) die virusartigen Partikel von dem abgetrennten Adsorptionsmittel desorbiert werden, so dass sie in einen Überstand einer dabei gebildeten zweiten Suspension gelangen und
f) der die virusartigen Partikel enthaltende Überstand von der zweiten Suspension abgetrennt wird,
wobei anschließend die virusartigen Partikel aus dem abgetrennten Überstand abgetrennt und gereinigt werden,
**dadurch gekennzeichnet,**
**dass** im Schritt e) zum Desorbieren der virusartigen Partikel ein Desoxycholsäure und/oder wenigstens ein lösliches Salz der Desoxycholsäure enthaltender Desorptionspuffer zugegeben wird und
**dass** nach dem Schritt f):
g) dem abgetrennten Überstand ein lösliches Calciumsalz zugegeben wird, wobei ein ein Calciumsalz der Desoxycholsäure und die virusartigen Partikel enthaltender Niederschlag gebildet wird,
h) der gebildete Niederschlag abgetrennt und in eine dritte Suspension überführt wird und
i) die virusartigen Partikel aus der dritten Suspension abgetrennt und gereinigt werden.

2. Verfahren nach Anspruch 1, **dadurch gekennzeichnet, dass** im Schritt e) zum Desorbieren der virusartigen Partikel ein Desorptionspuffer zugegeben wird, der die Desoxycholsäure bzw. das lösliche Salz der Desoxycholsäure in einer Stoffmengenkonzentration zwischen 1 mmol/l und 10 mmol/l, vorzugsweise etwa 6 mmol/l, enthält.

3. Verfahren nach Anspruch 2, **dadurch gekennzeichnet, dass** im Schritt g) dem abgetrennten Überstand das lösliche Calciumsalz in einer Stoffmengenkonzentration zwischen 30 mmol/l und 100 mmol/l, vorzugsweise zwischen 40 mmol/l und 60 mmol/l, zugegeben wird.

4. Verfahren nach Anspruch 2 oder 3, **dadurch gekennzeichnet, dass** im Schritt e) zum Desorbieren der virusartigen Partikel von dem Adsorptionsmittel der die Desoxycholsäure und/oder das lösliche Salz der Desoxycholsäure enthaltende Desorptionspuffer in einer Menge zugegeben wird, die dem 0,1-fachen bis 4-fachen des Volumens des im Schritt b) abgetrennten Überstands entspricht.

5. Verfahren nach einem der Ansprüche 1 - 4, **dadurch gekennzeichnet, dass** im Schritt g) dem abgetrennten Überstand Calciumchlorid zugegeben wird.

6. Verfahren nach einem der Ansprüche 1 - 5, **dadurch gekennzeichnet, dass** im Schritt c) dem abgetrennten Überstand ein nichtionisches Tensid zugemischt wird, so dass eine Konzentration des nichtionischen Tensids zwischen 0,006 g/ml und 0,01 g/ml, vorzugsweise von etwa 0,008 g/ml, eingestellt wird.

7. Verfahren nach einem der Ansprüche 1 - 6, **dadurch gekennzeichnet, dass** im Schritt d) das Adsorptionsmittel mit den adsorbierten virusartigen Partikeln abgetrennt wird, wobei das Adsorptionsmittel mit den adsorbierten virusartigen Partikeln gewaschen wird, wobei der Waschlösung Harnstoff zugegeben wird.

8. Verfahren nach Anspruch 7, **dadurch gekennzeichnet, dass** das Adsorptionsmittel mit den adsorbierten virusartigen Partikeln gewaschen wird, indem in einem ersten Waschschritt der Waschlösung Harnstoff in einer Stoffmengenkonzentration zwischen 2 mol/l und 6 mol/l, vorzugsweise etwa 4 mol/l, zugegeben wird und in einem zweiten Waschschritt mit einer wässrigen Natriumchloridlösung gewaschen wird.

9. Verfahren nach einem der Ansprüche 1 - 8, **dadurch gekennzeichnet, dass** im Schritt h) der gebildete Niederschlag in eine dritte Suspension überführt wird, indem der Niederschlag in einer schwach basischen Pufferlösung mit einem pH-Wert zwischen 7,5 und 8,5 aufgelöst wird.

10. Verfahren nach einem der Ansprüche 1 - 9, **dadurch gekennzeichnet, dass** im Schritt i) die virusartigen Partikel aus der dritten Suspension mit Hilfe eines chromatographischen Verfahrens abgetrennt werden.

11. Verfahren nach Anspruch 10, **dadurch gekennzeichnet, dass** als chromatographisches Verfahren eine Anionen-Austausch-Chromatographie durchgeführt wird, wobei dem Eluenten ein Tensid zugemischt wird.

12. Verfahren nach Anspruch 11, **dadurch gekennzeichnet, dass** im Schritt i) dem Eluenten als Tensid Polysorbat 20 zugemischt wird.

13. Verfahren nach Anspruch 10, **dadurch gekennzeichnet, dass** als chromatographisches Verfahren eine multimodale Chromatographie durchgeführt wird.

14. Verfahren nach einem der Ansprüche 1 - 13, **dadurch gekennzeichnet, dass** die virusartigen Partikel ein Hüllprotein aufweisen, das zumindest einen Abschnitt umfasst, der dem kleinen Hüllprotein (s-dHBsAg) eines Enten-Hepatitis-B-Virus oder dem kleinen Hüllprotein (s-HBsAg) eines humanen Hepatitis-B-Virus entspricht.

15. Verfahren nach einem der Ansprüche 1 - 14, **dadurch gekennzeichnet, dass** die virusartigen Partikel ein Hüllprotein aufweisen, das ein Hybridprotein mit einem dem kleinen Hüllprotein eines Virus der Familie Hepadnaviridae entsprechenden Abschnitt und wenigstens einem ein Zielepitop bildenden Abschnitt ist.

16. Verfahren nach Anspruch 15, **dadurch gekennzeichnet, dass** das Hybridprotein wenigstens zwei jeweils ein Zielepitop bildende Abschnitte aufweist.

17. Verfahren nach Anspruch 15 oder 16, **dadurch gekennzeichnet, dass** das wenigstens eine Zielepitop einem Epitop eines Virus einer Gruppe entspricht, die Bovine Virusdiarrhoe-Viren, West-Nil-Viren und Schweinepest-Viren umfasst.

18. Verfahren nach einem der Ansprüche 1 - 17, **dadurch gekennzeichnet, dass** die Wirtszellen Zellen eines rekombinanten Hefestamms der Ordnung Saccharomycetales (Echte Hefen) oder der Gattung Schizosaccharomyces (Spalthefen) sind.

19. Verfahren nach Anspruch 18, **dadurch gekennzeichnet, dass** die Wirtszellen Zellen eines rekombinanten Stamms der *Ogataea angusta* (*Hansenula polymorpha*) sind.

## Claims

1. A method for separating virus-like particles from a cell suspension of host cells containing the virus-like particles, wherein the virus-like particles have at least one envelope protein embedded in a lipid double membrane, the envelope protein comprising at least a portion corresponding to a small envelope protein of a virus of the family Hepadnaviridae, wherein
a) the host cells are disrupted in order to obtain a first suspension containing the virus-like particles,
b) a supernatant containing the virus-like particles is separated from the first suspension,
c) an adsorbent is added to the supernatant separated from the first suspension such that the virus-like particles are adsorbed by the adsorbent,
d) the adsorbent with the adsorbed virus-like particles is separated off,
e) the virus-like particles are desorbed from the separated-off adsorbent, so that they pass into a supernatant of a second suspension formed thereby, and
f) the supernatant containing the virus-like particles is separated from the second suspension,
wherein, subsequently, the virus-like particles are separated from the separated-off supernatant and purified,
**characterized in that**
in step e), for desorbing the virus-like particles, a desorption buffer is added which contains deoxycholic acid and/or at least one soluble salt of deoxycholic acid, and
after step f):
g) a soluble calcium salt is added to the separated-off supernatant, forming a precipitate containing a calcium salt of deoxycholic acid and the virus-like particles,
h) the precipitate formed is separated off and transferred to a third suspension and
i) the virus-like particles are separated from the third suspension and purified.

2. The method according to claim 1, **characterized in that**, in step e), for desorbing the virus-like particles, a desorption buffer is added, which contains the deoxycholic acid and/or the soluble salt of deoxycholic acid, respectively, in a molar concentration between 1 mmol/l and 10 mmol/l, preferably about 6 mmol/l.

3. The method according to claim 2, **characterized in that**, in step g), the soluble calcium salt is added to the separated-off supernatant in a molar concentration between 30 mmol/l and 100 mmol/l, preferably between 40 mmol/l and 60 mmol/l.

4. The method according to claim 2 or 3, **characterized in that**, in step e), for desorbing the virus-like particles from the adsorbent, the desorption buffer containing the deoxycholic acid and/or the soluble salt of deoxycholic acid, respectively, is added in an amount of 0.1 times to 4 times the volume of the supernatant separated off in step b).

5. The method according to any of claims 1 - 4, **characterized in that** calcium chloride is added to the separated-off supernatant in step g).

6. The method according to any of claims 1 - 5, **characterized in that**, in step c), a nonionic surfactant is added to the separated-off supernatant, so that a concentration of the nonionic surfactant between 0.006 g/ml and 0.01 g/ml, preferably of about 0.008 g/ml, is adjusted.

7. The method according to any of claims 1 - 6, **characterized in that**, in step d), the adsorbent with the adsorbed virus-like particles is separated off, wherein the adsorbent with the adsorbed virus-like particles is washed, wherein urea is added to the wash solution.

8. The method according to claim 7, **characterized in that** the adsorbent with the adsorbed virus-like particles is washed by adding urea to the wash solution in a molar concentration between 2 mol/l and 6 mol/l, preferably about 4 mol/l, in a first washing step and washing with an aqueous sodium chloride solution in a second washing step.

9. The method according to any of claims 1 - 8, **characterized in that**, in step h), the precipitate formed is transferred to a third suspension by dissolving the precipitate in a weakly basic buffer solution having a pH value between 7.5 and 8.5.

10. The method according to any of claims 1 - 9, **characterized in that**, in step i), the virus-like particles are separated from the third suspension by means of a chromatographic method.

11. The method according to claim 10, **characterized in that** an anion exchange chromatography is carried out as the chromatographic method, wherein a surfactant is added to the eluent.

12. The method according to claim 11, **characterized in that**, in step i), polysorbate 20 is added to the eluent as a surfactant.

13. The method according to claim 10, **characterized in that** a multimodal chromatography is performed as the chromatographic method.

14. The method according to any of claims 1 - 13, **characterized in that** the virus-like particles have an envelope protein comprising at least a portion which corresponds to the small envelope protein (s-dHBsAg) of a duck hepatitis B virus or the small envelope protein (s-HBsAg) of a human hepatitis B virus.

15. The method according to any of claims 1 - 14, **characterized in that** the virus-like particles have an envelope protein, which is a hybrid protein with a portion corresponding to the small envelope protein of a virus of the family Hepadnaviridae and at least one portion forming a target epitope.

16. The method according to claim 15, **characterized in that** the hybrid protein has at least two portions, each forming a target epitope.

17. The method according to claim 15 or 16, **characterized in that** the at least one target epitope corresponds to an epitope of a virus of a group comprising bovine viral diarrhea viruses, West Nile viruses and swine fever viruses.

18. The method according to any of claims 1 - 17, **characterized in that** the host cells are cells of a recombinant yeast strain of the order Saccharomycetales (real yeasts) or the genus Schizosaccharomyces (fission yeasts).

19. The method according to claim 18, **characterized in that** the host cells are cells of a recombinant strain of *Ogataea angusta* (*Hansenula polymorpha*).

## Revendications

1. Procédé de séparation de particules viroïdes d'une suspension cellulaire de cellules hôtes contenant les particules viroïdes, les particules viroïdes comportant au moins une protéine d'enveloppe incorporée dans une double membrane lipidique, qui comporte au moins un segment qui correspond à une petite protéine d'enveloppe d'un virus de la famille des hépadnavirus (Hepadnaviridae), lors duquel
a) l'on ouvre les cellules hôtes pour obtenir une première suspension contenant les particules viroïdes,
b) l'on sépare de la première suspension un surnageant contenant les particules viroïdes,
c) l'on ajoute au surnageant séparé de la première suspension un agent adsorbant, de telle sorte que les particules viroïdes soient adsorbées par l'agent adsorbant,
d) l'on sépare l'agent adsorbant avec les particules viroïdes adsorbées,
e) l'on désorbe les particules viroïdes de l'agent adsorbant séparé, de sorte qu'elles arrivent dans un surnageant d'une deuxième suspension créée à cet effet et
f) l'on sépare le surnageant contenant les particules viroïdes de la deuxième suspension,
lors duquel l'on sépare par la suite les particules viroïdes du surnageant séparé et on les purifie, **caractérisé**
**en ce que** dans l'étape e), pour désorber les particules viroïdes, l'on ajoute un tampon de désorption contenant l'acide désoxycholique et/ou au moins un sel soluble de l'acide désoxycholique et
**en ce qu'**après l'étape f) :
g) l'on ajoute au surnageant séparé un sel de calcium soluble, un précipité contenant un sel de calcium de l'acide désoxycholique et les particules viroïdes étant créé,
h) l'on sépare le précipité séparé et on le transfère dans une troisième suspension et
i) l'on sépare les particules viroïdes de la troisième suspension et on les purifie.

2. Procédé selon la revendication 1, **caractérisé en ce que** dans l'étape e), pour désorber les particules viroïdes, on ajoute un tampon de désorption qui contient l'acide désoxycholique ou le sel soluble de l'acide désoxycholique dans une concentration molaire comprise entre 1 mmole/1 et 10 mmoles/1, de préférence d'environ 6 mmoles/1.

3. Procédé selon la revendication 2, **caractérisé en ce que** dans l'étape g), l'on ajoute au surnageant séparé le sel de calcium soluble dans une concentration molaire comprise entre 30 mmoles/1 et 100 mmoles/1, de préférence comprise entre 40 mmoles/1 et 60 mmoles/1.

4. Procédé selon la revendication 2 ou 3, **caractérisé en ce que** dans l'étape e), pour désorber les particules viroïdes de l'agent adsorbant, l'on ajoute le tampon de désorption contenant l'acide désoxycholique et/ou le sel soluble de l'acide désoxycholique dans une quantité qui correspond à 0,1 fois à 4 fois le volume du surnageant séparé dans l'étape b).

5. Procédé selon l'une quelconque des revendications 1 à 4, **caractérisé en ce que** dans l'étape g), l'on ajoute du chlorure de calcium au surnageant séparé.

6. Procédé selon l'une quelconque des revendications 1 à 5, **caractérisé en ce que** dans l'étape c), l'on mélange au surnageant séparé un agent tensio-actif non ionique, de sorte à régler une concentration de l'agent tensio-actif non ionique comprise entre des 0,006 g/ml et 0,01 g/ml, de préférence d'environ 0,008 g/ml.

7. Procédé selon l'une quelconque des revendications 1 à 6, **caractérisé en ce que** dans l'étape d), l'on sépare l'agent adsorbant avec les particules viroïdes adsorbées, l'agent adsorbant avec les particules viroïdes adsorbées étant lavé, de l'urée étant ajoutée à la solution de lavage.

8. Procédé selon la revendication 7, **caractérisé en ce que** l'on lave l'agent adsorbant avec les particules viroïdes adsorbées, **en ce que** dans une première étape de lavage, l'on ajoute à la solution de lavage de l'urée dans une concentration molaire comprise entre 2 moles/1 et 6 moles/1, de préférence d'environ 4 moles/1, et dans une deuxième étape de lavage, on lave avec une solution aqueuse de chlorure de sodium.

9. Procédé selon l'une quelconque des revendications 1 à 8, **caractérisé en ce que** dans l'étape h), l'on transfère le précipité créé dans une troisième suspension, **en ce qu'**on dilue le précipité dans une solution tampon faiblement basique, ayant une valeur pH comprise entre 7,5 et 8,5.

10. Procédé selon l'une quelconque des revendications 1 à 9, **caractérisé en ce que** dans l'étape i), l'on sépare les particules viroïdes de la troisième suspension à l'aide d'un procédé chromatographique.

11. Procédé selon la revendication 10, **caractérisé en ce que** l'on réalise en tant que procédé chromatographique une chromatographie d'échange anionique, un agent tensio-actif étant mélangé à l'éluant.

12. Procédé selon la revendication 11, **caractérisé en ce que** dans l'étape i), l'on mélange à l'éluant en tant qu'agent tensio-actif du polysorbate 20.

13. Procédé selon la revendication 10, **caractérisé en ce que** l'on réalise en tant que procédé chromatographique une chromatographie multimodale.

14. Procédé selon l'une quelconque des revendications 1 à 13, **caractérisé en ce que** les particules viroïdes comportent une protéine d'enveloppe qui comprend au moins un segment qui correspond à la petite protéine d'enveloppe (s-dHBsAg) d'un virus de l'hépatite B chez le canard ou à la petite protéine d'enveloppe (s-HBsAg) d'un virus de l'hépatite B chez l'humain.

15. Procédé selon l'une quelconque des revendications 1 à 14, **caractérisé en ce que** les particules viroïdes comportent une protéine d'enveloppe qui est une protéine hybride avec un segment correspondant à la petite protéine d'enveloppe d'un virus de la famille des hépadnavirus (Hepadnaviridae) et au moins un segment formant un épitope cible.

16. Procédé selon la revendication 15, **caractérisé en ce que** la protéine hybride comporte au moins deux segments formant chacun un épitope cible.

17. Procédé selon la revendication 15 ou 16, **caractérisé en ce que** l'au moins un épitope cible correspond à un épitope d'un virus d'un groupe comprenant les virus de la diarrhée virale bovine, les virus du Nil occidental et les virus de la peste porcine.

18. Procédé selon l'une quelconque des revendications 1 à 17, **caractérisé en ce que** les cellules hôtes sont des cellules d'une souche de levure recombinante de l'ordre des Saccharomycetales (levures bourgeonnantes) ou de l'espèce des Schizosaccharomyces (levures de fission).

19. Procédé selon la revendication 18, **caractérisé en ce que** les cellules hôtes sont des cellules d'une souche recombinante des Ogataea angusta (Hansenula polymorpha).
